(19) Europäisches Patentamt
European Patent Office
Office européen des brevets

(11) Publication number: **0 095 884**
**B1**

(12) # EUROPEAN PATENT SPECIFICATION

(45) Date of publication of patent specification: **23.09.87**

(51) Int. Cl.$^4$: **A 01 N 37/20**

(21) Application number: **83302980.4**

(22) Date of filing: **24.05.83**

(54) **Improvements in or relating to quaternary ammonium compound disinfectants for use in bovine mastitis control.**

(30) Priority: **25.05.82 GB 8215204**

(43) Date of publication of application:
**07.12.83 Bulletin 83/49**

(45) Publication of the grant of the patent:
**23.09.87 Bulletin 87/39**

(84) Designated Contracting States:
**AT BE CH DE FR IT LI LU NL SE**

(56) *References cited:*
**GB-A-1 554 615**
**US-A-3 993 777**
**US-A-4 069 347**

**SOAP/COSMETICS/CHEMICAL SPECIALTIES, vol. 52, no. 10, October 1976, pages 33,34,38,40, New York, USA M.L. SCHLOSSMANN: "Quaternized Lanolin in *Cosmetics*"**

**The file contains technical information submitted after the application was filed and not included in this specification**

(73) Proprietor: **Wilson, Edward Sturdy**
**Altahammond 57 Belfast Road**
**Whitehead County Antrim Northern Ireland (GB)**

(72) Inventor: **Wilson, Edward Sturdy**
***Altahammond* 57 *Belfast Road***
**Whitehead County Antrim Northern Ireland (GB)**

(74) Representative: **Kyle, Diana**
**ELKINGTON AND FIFE High Holborn House**
**52/54 High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 095 884 B1

**Description**

This invention relates to quaternary ammonium compound disinfectant compositions suitable for topical application and which have inherent emollient and humectant properties and, in particular, to teat dips for use in the prevention of bovine mastitis.

Certain quaternary ammonium compounds (Q.A.C's) are known to be powerful antiseptics. These quaternary ammonium compounds include cetyltrimethylammonium chloride and cetylpyridinium chloride. It is known to use one or more Q.A.C's in aqueous solution as teat dips in bovine mastitis control, such Q.A.C.'s include benzalkonium chloride and cetyltrimethylammonium chloride. However, if the Q.A.C. or Q.A.C.'s is/are used merely in the form of an aqueous solution, two undesirable side effects arise:

a) the aqueous solvent evaporates at body temperature leaving a thin concentrated film of the Q.A.C. or Q.A.C's on the teat surface. The residual film is irritant to the skin and can cause severe rubefaction; and

b) since Q.A.C.'s are powerful surfactants, in daily use they emulsify the natural fat of the skin which eventually leads to loss of dermal flexibility resulting in teat cracking.

Side effects a) and b) have been largely and successfully alleviated by the compositions of the Applicant's British Patent Specification No. 1554615 which compositions include in addition to one or more Q.A.C.(s) a humectant, which prevents the complete evaporation of water from the teat after dipping, and an emollient which is soluble or emulsifiable in water. In the compositions of British Patent Specification No. 1554615 the humectant ensures that the amount of water which can evaporate at the body temperature of the teat is controlled within limits, such that a concentrated film of Q.A.C.(s) is prevented from forming on the teat. The emollient, which is selected from emollients such as ethoxylated lanolin, synthetic plumage oil and ethoxylated synthetic plumage oil as hereinafter defined, counteracts emulsification of the skin fats by the Q.A.C's and this prevents teat cracking.

It is an object of the present invention to provide a quaternary ammonium compound disinfectant composition which is particularly suitable for use as a teat dip and which has inherent emollient and humectant activity.

Accordingly, the invention provides use of a compound of the general formula I:

$$\left[ L{-}NR_1{-}(CH_2)_n{-}\overset{\displaystyle R_2}{\underset{\displaystyle R_4}{\overset{|}{\underset{|}{N^{\oplus}}}}}{-}R_3 \right] X^{\ominus} \qquad I$$

wherein L represents an acyl radical derived from lanolin fatty acid, $R_1$ is hydrogen or a $C_{1-4}$ alkyl group, $R_2$ and $R_3$ each represents a $C_{1-4}$ alkyl group, $R_4$ is an alkyl, aralkyl, hydroxyalkyl or unsaturated aliphatic hydrocarbon radical, X is a compatible anion and n is an integer of from 2 to 5, for the manufacture of a disinfectant composition for teat-dipping.

Preferably, the active ingredient is a compound of formula I wherein $R_1$ is hydrogen, $R_2$ and $R_3$ independently of each other represent a methyl or ethyl group, $R_4$ is a $C_{1-4}$ alkyl group or an ar($C_{1-4}$) alkyl group, X is a compatible anion and n=2 or 3. Preferably, X is a halide, nitrate, sulphate, alkylsulphate or alkylphosphate anion.

Most preferably, L represents an acyl radical derived from refined lanolin acid.

An especially preferred compound of formula I is one wherein L is an acyl radical derived from a refined lanolin fatty acid, $R_1$ is hydrogen, $R_2$ and $R_4$ are each methyl, $R_3$ is ethyl, n=3 and X is an ethylsulphate ion *viz* lanolinamidopropyldimethylethylammonium ethosulphate.

Commercially available lanolin Q.A.C.'s are primarily used in the cosmetics industry and tend to be made and used in diol (glycol) solution, for example in propylene glycol. British Patent Specification No. 1590012 discloses compositions for use in cosmetic formulations, especially hair care preparations, which comprise a mixture of at least one lanolin Q.A.C. of the formula I above and at least one aliphatic, saturated, branched-chain diol having from 5—20 carbon atoms.

Many of the lanolin Q.A.C.'s useful as active ingredient in the compositions manufactured using a compound of general formula I according to the invention have a consistency which is similar to that of petroleum jelly. With such lanolin Q.A.C.'s it has been found that it is easier to prepare a composition according to the invention if the Q.A.C. is used in conjunction with a non-ionic surfactant. Any commercially available non-ionic surfactant is suitable. However, a particularly suitable non-ionic surfactant is TEXOFOR V27 (TEXOFOR V27 is a trade mark) which is a polyoxyalkylene condensate of a linear fatty alcohol. Other suitable non-ionic surfactants are (i) the non-ionics referred to as polyoxyethylene derivatives of sorbitan fatty acid esters or alkyl sorbitan polyoxyethylene marketed under the trade mark "Tweens", the most preferred being "Tween 80", also known as polysorbate 80 or sorbitan mono-oleate; and (ii) the non-ionics referred to as polyoxyethylene-polyoxypropylene block polymers marketed under the trade mark "Pluronics", particularly those having molecular weights ranging from 4,000 to 8,000 with approximately 40—70% of the polyoxyethylene hydrophilic polymer and 60—30%, respectively, of the polyoxypropylene hydrophobic polymer, the most preferred being "Pluronic F127", also known as poloxomer 407. Preferably, the lanolin Q.A.C.(s) will be used in an amount of 25—50% by

weight and the non-ionic surfactant in an amount of 50—75% by weight of the lanolin Q.A.C.(s)/surfactant mixture.

The compounds of formula I are known and can be readily prepared by conventional techniques such as by reaction of a lanolin acid of formula II:

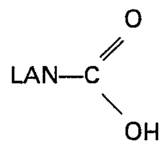

II

wherein LAN represents the fatty acid side chain of a lanolin acid, with a diamine having the formula III:

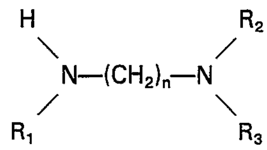

III

wherein $R_1$ is hydrogen or a $C_{1-4}$ alkyl group, $R_2$ and $R_3$ are $C_{1-4}$ alkyl groups and n is an integer from 2 to 5 to form a lanolin amide of formula IV:

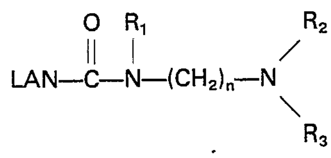

IV

The lanolin amide of formula IV is treated with a suitable quaternizing agent such as an aliphatic halide, aralkyl halide, ethylene chlorohydrin or alkyl sulphate so as to obtain the corresponding compound of formula I.

Especially preferred quaternizing agents include alkyl halides such as methyl bromide, ethyl iodide or isopropyl bromide, aralkyl halides such as benzyl chloride, alkyl sulphates such as dimethyl or diethyl sulphate or ethylene chlorohydrin. However, any suitable known quaternizing agent can be used.

The lanolin acid of formula II may be any commercially available lanolin fatty acid or mixture of, such lanolin fatty acids.

Representative diamines of formula III for preparing preferred compounds of formula IV which are then quaternised to form the corresponding compounds of formula I include: dimethylaminopropylamine, diethylaminopropylamine, dimethylaminoethylamine and diethylaminoethylamine.

It will be appreciated from the foregoing description that the compositions according to the invention may comprise a mixture of compounds of formula I.

Preferably, the total concentration of the compound(s) of formula I in the compositions according to the invention is in the range 10,000—50,000 p.p.m. more particularly, 10,000—40,000 p.p.m. and especially about 20,000 p.p.m.

Aqueous solutions of the compounds of formula I have both emollient and antiseptic properties due to the bifunctional nature of the molecules of the compounds of formula I, unlike aqueous solutions of Q.A.C.'s conventionally used as disinfecting compositions in the prevention of bovine mastitis.

The disinfectant compositions manufactured using a compound of general formula (I) according to the invention are used as teat or udder dips (hereinafter referred to as teat dips) for cows for the prevention of mastitis.

The criterion to be achieved in any teat dip solution is an active ingredient having a high degree of biocidal activity such as that exhibited by the compounds of formula I. However, equally important is the skin care of the teats.

Accordingly, despite the inherent emolliency of the compounds of formula I, the compositions according to the invention preferably include at least one emollient, soluble or emulsifiable in water, at a concentration in the range 0.1—1.0% by weight of the composition, especially in the range 0.25%—0.75% by weight of the composition.

Preferred emollients are selected from: alkoxylated lanolin, especially ethoxylated lanolin, synthetic plumage oil, and ethoxylated synthetic plumage oil.

Synthetic plumage oil, as the term is used in this Specification, is a synthetic equivalent of the oil which occurs on the plumage of water fowl namely preen gland oil (oleum glandulae uropygialis). Synthetic plumage oil is a highly branched long chain fatty ester having an even numbered (about $C_{18}$) carbon atom chain in the acid portion of the molecule, or more usually a mixture of such esters. It can be cooled and filtered to yield a liquid and a solid product, sold respectively under the names PCL liquid and PCL Solid; both are insoluble in water.

PCL Liquid if mixed with a substantially equal volume of a non-ionic surfactant will form a transparent colloidal solution in water. A suitable PCL/surfactant mixture of this kind is sold under the name Neo PCL water soluble 2/966212 or simply as Neo PCL; the surfactant in this mixture is itself a mixture of ethoxylated octyl and nonyl phenols which is sold separately under the name LISSAPOL (Registered Trade Mark) and EMPILAN (Registered Trade Mark) and contains about 14 moles of ethylene oxide residue per mole of alkyl phenol. Alternatively, a conventional emulsifying agent can be used to emulsify an insoluble emollient, such as PCL Liquid, in the compositions according to the invention.

Ethoxylated synthetic plumage oil is manufactured by treatment of synthetic plumage oil with ethylene oxide. Ethoxylated synthetic plumage oil containing about 14 molar equivalents of ethoxyl group per mole of synthetic plumage oil and which is freely soluble in water is commercially sold under the trade name Ethoxylated PCL.

The products hereinabove referred to by the trade names under which they are sold are available from the Dragoco Company of Dusseldorf, Germany.

An especially preferred teat dip according to the invention is one containing approximately 0.25% by weight of Neo-PCL water soluble 2/966212—hereinafter referred to as Neo PCL. Neo PCL is fully compatible with the compounds of formula I. The addition of Neo PCL to a compound of formula I results in an increase in viscosity. The properties and activity of Neo PCL in teat dips according to the invention is discussed below.

The biocidal activity of the compositions according to the invention can be enhanced by incorporating therein one or more quaternary ammonium halide compounds at a concentration in the range 0.010—2.0%, more particularly 0.1—1.0% by weight of the composition. Especially preferred quaternary ammonium halides are quaternary ammonium chlorides such as benzalkonium chloride, octyl decyl dimethyl ammonium chloride, dioctyl dimethyl ammonium chloride or didecyl dimethyl ammonium chloride. Benzalkonium chloride is especially preferred at a concentration of 0.01% by weight of the composition.

The ratio of the compound(s) of formula I to the quaternary ammonium halide compound(s) should not be less than 3:1.

The teat dips according to the invention have been found to be superior in terms of biocidal activity and skin care to the teat dips of the Applicant's British Patent Specification No. 1554615.

Although not wishing to be bound by an theoretical explanation of the mode of action of the compositions according to the invention, it is my belief that the following explanation will be generally accepted by those skilled in the art. It is known that all Q.A.C's are ionically active being cationic surfactants and as such have a high affinity for oppositely charged substrates and, in particular, for largely proteinaceous substances such as hair, wool and skin. It is this affinity, coupled with concentration of the Q.A.C., due to evaporation in use in the case of known teat dips comprising conventionally used Q.A.C.'s, such as quaternary ammonium halides, which causes rubefaction.

As stated above, in the case of the Q.A.C. compositions of the Applicant's British Patent Specification No. 1554615 rubefaction and teat cracking are combated by the inclusion of a separate humectant and a separate emollient material.

In the case of the compositions of the present invention one has the ideal theoretical combination of a biocidal Q.A.C. and an emollient all in one bifunctional molecule rather than in a mixture. Accordingly, the Q.A.C. will always be in intimate association with a lanolin material since a lanolin moiety forms part of the compounds of formula I used in the compositions according to the invention.

The inclusion of an emollient such as Neo PCL in the compositions according to the invention is to provide additional skin care. In combination with the compounds of formula I the emollient acts to nourish the teat skin and keep it supple. The emollient and the quaternary ammonium compound of formula I, which is a lanolin derivative, in combination keep the teat skin from being degreased by udder washing and teat dipping, leading to a healthy, pliable teat skin capable of withstanding the demands of many lactations.

Stripping of the surface sebum of the teat deprives the teat skin of its smoothness and exposes it to penetration by dirt particles and microorganisms. Furthermore, removal of the sebum brings with it the risk of drying of the epidermis. The suppleness and mechanical strength of the stratum corneum is determined by its water content.

The inclusion of Neo PCL, which is a synthetic analogue of the surface sebum, combats any stripping of the surface sebum from the teat skin.

It has been found that Neo PCL is highly diffusible and results in complete coverage of the teat skin surface with at least a monomolecular layer of Neo PCL. Neo PCL has a marked effect on the diffusion of the active ingredient of formula I by causing an increased rate of diffusion resulting in greater protection due to a more complete coverage of the teats with the active ingredient.

The low melting point of Neo PCL resembles the glycerides of unsaturated fatty acids which are also present in animal sebum. However, Neo PCL is a fully saturated compound which is not liable to oxidation on the teat skin. Neo PCL is not affected by the action of u.v. radiation on the teat skin surface when the teat skin is exposed to sunlight. Accordingly, teat dips according to the invention and which include Neo PCL are particularly suitable for use in tropical and sub-tropical environments.

Straight chain fatty esters are also prone to decomposition on the teat skin by enzyme action, notably

by hydrolysis by an esterase enzyme which is normally present on the skin surface, resulting in the release of free fatty acids. Neo PCL has been found to be highly resistant to this form of chemical breakdown.

Neo PCL when present in teat dips according to the invention will counteract any extensive degreasing of the skin by udder washing. As the skin of bovine teats is bland, completely lacking in sebaceous glands and hair follicles, the Neo PCL can achieve a degree of fat restoration a) by simple reduction of the degreasing effect of detergent materials, b) by partial bonding of the surfactant molecules, or c) by deposition of the super fatting agent on the skin tissue. It is likely that effects a)-c) occur together. In experiments carried out to demonstrate the ability of Neo PCL to restore fat to skin tissue in the presence of a surfactant, the PCL Liquid settles out of an aqueous solution on to absorbent cotton wads.

An important characteristic of Neo PCL is the absence of what is known as the occlusive effect when it is used as an emollient in teat dips. Healthy bovine teat skin continuously and simultaneously releases water vapour and carbon dioxide to the atmosphere and takes up oxygen. An unrestricted exchange of the latter substances with the environment, most notably the free evaporation of water, is a necessary condition for the physiological equilibrium of the teat skin. Restriction of water evaporation leads to hyperhydration of the stratum corneum, making it softer and less resistant to the intrusion of foreign bodies. Hyper-hydration also raises the skin temperature. These changes at the skin surface can have undesirable secondary effects, for example hyper-hydration caused by occlusion brings about an increase in the bacterial count on the skin surface and qualitative changes in the skin flora. Emollients which cover the skin by occlusion risk impairment of the normal exchange of carbon dioxide, water vapour and oxygen with the environment. In nature, occlusion is prevented by the presence of branched chain fatty acids and this characteristic has been demonstrated with preen gland oil of the duck; these branched chain fatty acids also occur in animal sebum. Neo PCL consists mainly of esters of branched chain fatty acids and would therefore be expected to have the same effect as preen gland oil.

In the teat dips according to the invention, Neo PCL is superior to other lipids used as emollients in teat dip solutions in its permeability to water vapour, which is similar to the action of the thin film of fatty acids naturally present on the teat skin.

In conclusion, the advantages of using Neo PCL in the compositions according to the invention are its outstanding spreadibility and affinity for the skin. Neo PCL exhibits only slight variations in viscosity with temperature, is stable to oxidation and esterase action, promotes the uptake of the active ingredient of the compositions according to the invention and is compatible with surfactants.

Neo PCL is readily incorporated in the compositions according to the invention and meets the standard criteria of dermatological and toxicological safety as it is widely used in human cosmetic preparations.

The following Examples illustrate the preparation of compositions using a compound of general formula I according to the invention. The Preparations illustrate the preparation of Q.A.C.'s for use as the active ingredient in disinfectant compositions.

Preparation 1

Lanolinamidopropyldimethylbenzylammonium chloride

(a) Dimethylaminopropyl lanolin acid amide

One mole (about 340 grams) of warn lanolin fatty acids was poured into a flask which was fitted with a stirrer, thermometer, dropping funnel and reflux condenser. The air in the apparatus was displaced with nitrogen and the contents heated within the range 105—115°C.

Over the next 15 to 20 minutes 130 grams (about 1.3 moles) of dimethylaminopropylamine were added dropwise and the temperature increased to 140°C to 150°C. This temperature range was maintained for some 7 to 8 hours. During the final 4 hours a vacuum was applied which it was possible to reduce to 1 mm Hg during the last hour when the temperature was raised to 160°C.

The reaction mixture was thereafter cooled to yield the amide of the original lanolin fatty acids.

(b) Quaternization of the dimethylaminopropyl lanolin acid amide

About 200 grams of the dimethylaminopropyl lanolin acid amide were placed in a reaction vessel and heated to 75°C. At this point 62 grams of benzyl chloride were dropped in slowly over a period of 1 hour and the mixture was maintained at 110°C for 3 hours. During the last hour of the reaction the pressure was reduced as before. The mixture was cooled to yield the final product—the quaternary ammonium chloride of the lanolin fatty acids. The final product had the consistency of petroleum jelly and the characteristic odour of a Q.A.C. Analysis indicated *circa* 78% quaternization.

Preparation 2

Lanolinamidopropyldimethylethyl ammonium ethosulphate

Dimethylaminopropyl lanolin amide prepared according to Preparation 1—step (a)—was heated to 75°C in a reaction vessel and then an equivalent amount of diethylsulphate was added slowly over a period of one hour. The temperature was then raised to 110° and maintained at that temperature for 3 hours.

During the last half hour of the reaction the pressure was reduced to 1/mmHg.

The mixture was then cooled. The final product had a consistency of petroleum jelly and the characteristic odour of a Q.A.C. Analysis indicated *circa* 80% quaternization.

Example 1

The final product of Preparation 1 was mixed with a non-ionic surfactant TEXOFOR V27® in a ratio of 1:2.

A concentrated teat dip was prepared by dissolving the mixture of Q.A.C. and surfactant in water so as to give a Q.A.C. concentration of 30,000 p.p.m. by weight, by which is meant the total weight of the teat dip including the water.

Before use the concentrated teat dip (1 part) is diluted with water (2 parts) so as to obtain a final Q.A.C. concentration of 10,000 p.p.m.

Example 2

A concentrated teat dip was prepared by dissolving the product of Preparation 2 in water so as to give a Q.A.C. concentration in the range of 30,000 p.p.m. by weight by which is meant the total weight of the teat dip including the water as in the case of Example 1 above.

Before use the concentrated teat dip (1 part) is diluted with water (2 parts) so as to obtain a final Q.A.C. concentration of 10,000 p.p.m.

Example 3

A concentrated teat dip containing 30,000 p.p.m. of Q.A.C. was prepared in the same manner as the teat dip of Example 2 except that Neo PCL® was also added at a concentration of 0.25% by weight of the total weight of the teat dip.

Example 4

A concentrated teat dip containing 30,000 p.p.m. of Q.A.C. was prepared in the same manner as the teat dip of Example 2, except that benzalkonium chloride was also added at a concentration of 0.10%. Benzalkonium chloride B.P. was used which is a 50% solution. Accordingly, the benzalkonium solution was added at a concentration of 0.2% to achieve a final concentration of 0.10% in the teat dip.

Example 5

A concentrated teat dip was prepared as in the case of the teat dip of Example 1 except that Neo PCL® Liquid was included at a concentration of 0.25% by weight of the total weight of the teat dip.

Experimental field trials

A) The diluted teat dip of Example 1 was used in routine mastitis control in a small dairy herd over a period of 4 weeks at the morning and evening milking sessions. After 4 weeks it was found that no rubefaction had occurred. This would appear to be ample proof of the emollient action of the lanolin moiety of the compounds of formula I, since no other emollient was present in the teat dip.

B) The diluted teat dip of Example 2 was used in routine mastitis control in a small dairy herd over a period of 4 weeks at the morning and evening milking sessions. After 4 weeks it was found that no rubefaction had occurred. Again as in the case of Trial A) this would appear to be ample proof of the emollient action of the lanolin moiety of the compounds of formula I, since no other emollient was present in the teat dip.

C) The teat dip of Example 3 diluted with 2 parts of water was used in routine mastitis control in a small dairy herd over a period of 4 weeks at the morning and evening milking sessions with excellent results.

D) The teat dip of Example 4 diluted with 2 parts of water was used in routine mastitis control in a small dairy herd over a period of 2 weeks at the morning and evening milking sessions, with satisfactory results.

E) The teat dip of Example 5 diluted with 2 parts of water was used in routine mastitis control in a small dairy herd over a period of 4 weeks at the morning and evening milking sessions with excellent results.

Example 6

A teat dip was prepared from the following ingredients:

| | Concentration (% w/w) |
|---|---|
| Lanolinamidopropyldimethylethylammonium ethosulphate (Product of preparation 2) | 1.5 |
| TEXOFOR V27® | 1.5 |
| Neo PCL® liquid | 0.75 |
| Empigen BAC* | 1.0 |
| Water | 95.25 |

*BAC=benzalkonium chloride (Empigen BAC is a trade mark).

Activity of teat dip: 15,000 p.p.m. Q.A.C. ex. Lanolin Q.A.C.;
5,000 p.p.m. Q.A.C. ex. BAC

The concentrated solution (1 part) was then diluted with water (2 parts) to prepare a teat dip ready for use.

Activity of teat dip: 6,666 p.p.m. of total Q.A.C.

Example 7

A teat dip was prepared from the following ingredients:

| | Concentration (% w/w) |
|---|---|
| Lanolinamidopropyldimethylbenzylammonium chloride (Product of preparation 1) | 0.5 |
| TEXOFOR V27 ® | 0.5 |
| Neo PCL® liquid | 0.25 |
| Empigen BAC | 0.33 |
| Water | 98.42 |

The solution is ready for use as a teat dip without further dilution.

Activity of teat dip: 5,000 p.p.m. Q.A.C. ex. lanolin Q.A.C.;
1,600 Q.A.C. ex. Empigen BAC....
Total Q.A.C. 6,600 p.p.m.

Bactericidal efficiency of lanolinamidopropyldimethylethylammonium ethosulphate

Laboratory tests were carried out to determine the bactericidal efficiency of a lanolinamidopropyldimethylethylammonium ethosulphate teat dip against the following bacteria:

| | |
|---|---|
| 1. *Staphylococcus aureus* | NCTC 6571 |
| 2. *Escherichia coli* | NCTC 10418 |
| 3. *Pseudomonas aeruginosa* | NCTC 10662 |
| 4. *Streptococcus agalactiae* | Fermentation TYPE IV (V.R.L.) |

Method

Stock cultures of the bacteria were grown in Brain Heart Infusion broth (oxoid) incubated at 37°C for 18 hours. The stock cultures were diluted to give working suspensions containing approximately 10° cells per ml.

Bacterial concentrations of working suspensions and numbers of surviving bacteria after trial were estimated by culturing on Blood Agar Base No. 2 (Oxoid) supplemented with 7% Bovine serum (Oxoid). Trials were carried out in sterile test-tubes at a temperature of 37°C. Testing was carried out as follows:

Cultures of the test bacteria were exposed to a range of concentrations of lanolinamidopropyldimethylethylammonium ethosulphate (in a liquid test system) for a period of 10 minutes after which time the Q.A.C. was neutralised.

Inactivation of disinfectant after the required contact time was achieved using a neutralising solution containing 2.0% Lecithin, 2.0% Tween 80, 0.5% Sodium thiosulphate and 0.1% peptone.

The results are shown on Table 1.

The results show that in a liquid test system a concentration of at least 1.5% lanolinamidopropyldimethylethylammonium ethosulphate is required to effect a log 4 reduction in bacterial numbers during a 10 minute contact time at 37°C. Given the inherent statistical error in this type of analysis a concentration of greater than 1.5% is recommended to ensure an efficient kill against Pseudomonas aeruginosa which is noticeably more resistant to lanolinamidopropyldimethylethylammonium ethosulphate than are the other three test organisms.

TABLE 1

| Concentration lanolinamidopropyldimethylethylammonium ethosulphate | *Staphylococcus aureus* | *Escherichia coli* | *Pseudomonas aeruginosa* | *Streptococcus agalactiae* |
|---|---|---|---|---|
| 0.25% | 6.02 | 0.56 | 0.23 | 6.82 |
| 0.75% | 7.02 | 4.09 | 2.83 | 7.17 |
| 1.5% | 7.02 | 5.87 | 4.11 | 7.17 |
| 2.5% | 7.02 | 7.19 | 5.49 | 7.17 |

## Claims

1. Use of a compound of the general formula I:

$$\left[ L{-}NR_1{-}(CH_2)_n{-}\overset{\displaystyle R_2}{\underset{\displaystyle R_4}{\overset{|}{\underset{|}{N^{\oplus}}}}}{-}R_3 \right] X^{\ominus} \qquad I$$

wherein L represents an acyl radical derived from lanolin fatty acid, $R_1$ is hydrogen or a $C_{1-4}$ alkyl group, $R_2$ and $R_3$ each represents a $C_{1-4}$ alkyl group, $R_4$ is an alkyl, aralkyl, hydroxyalkyl or unsaturated aliphatic hydrocarbon radical, X is a compatible anion and n is an integer of from 2 to 5, for the manufacture of a disinfectant composition for teat-dipping.

2. Use of a compound according to claim 1, wherein in the general formula I, $R_1$ is hydrogen, $R_2$ and $R_3$ independently of each other represent a methyl or ethyl group, $R_4$ is a $C_{1-4}$ alkyl group or an ar($C_{1-4}$)alkyl group, X is a compatible anion and n is 2 or 3.

3. Use of a compound according to claim 1 or claim 2 wherein in the general formula I, X is a halide, especially chloride, nitrate, sulphate, alkylsulphate, especially ethylsulphate, or alkylphosphate anion and L represents an acyl radical derived from refined lanolin acid.

4. Use of a compound according to claim 1, wherein the disinfectant composition contains as an active ingredient a mixture of compounds of general formula I.

5. Use of a compound according to any one of claims 1—4, wherein the total concentration of the active compound(s) is in the range 10,000—50,000 p.p.m., especially 10,000—40,000 p.p.m., by weight of the composition.

6. Use of a compound according to any one of claims 1 to 5, wherein the disinfectant composition includes at least one emollient, soluble or emulsifiable in water, at a concentration in the range 0.1—1.0%, especially 0.25—0.75%, by weight of the composition.

7. Use of a compound according to claim 6, wherein the emollient is selected from alkoxylated lanolin, synthetic plumage oil (which is a highly branched long chain fatty ester having an even numbered carbon atom chain in the acid portion of the molecule or a mixture of such esters) and ethoxylated synthetic plumage oil as hereinbefore defined.

8. Use of a compound according to any one of claims 1—7, wherein the disinfectant composition includes one or more quaternary ammonium halide compounds at a concentration in the range 0.010—2.0% by weight of the composition, for increased biocidal activity.

9. Use of a compound according to claim 8, wherein the quaternary ammonium halide compound is selected from benzalkonium chloride, octyl decyl dimethyl ammonium chloride, dioctyl dimethyl ammonium chloride and didecyl dimethyl ammonium chloride.

## Patentansprüche

1. Verwendung einer Verbindung der allgemeinen Formel I

$$\left[ L{-}NR_1{-}(CH_2)_n{-}\overset{\displaystyle R_2}{\underset{\displaystyle R_4}{\overset{|}{\underset{|}{N^{\oplus}}}}}{-}R_3 \right] X^{\ominus} \qquad I$$

worin L eine Acylrest, der sich von Lanolinfettsäure ableitet, ist, $R_1$ Wasserstoff oder ein $C_{1-4}$-Alkylgruppe ist, $R_2$ und $R_3$ jeweils eine $C_{1-4}$-Alkylgruppe darstellen, $R_4$ ein Alkyl-, Aralkyl-, Hydroxyalkyl- oder ein ungesättigter aliphatischer Kohlenwasserstoffrest ist, X ein verträgliches Anion ist und n eine ganze Zahl von 2 bis 5 bedeutet, zur Herstellung einer Desinfektionszusammensetzung zum Eintauchen von Zitzen.

2. Verwendung einer Verbindung gemäß Anspruch 1, worin in der allgemeinen Formel I $R_1$ Wasserstoff ist, $R_2$ und $R_3$ unabhängig voneinander eine Methyl- oder Äthylgruppe darstellen, $R_4$ eine $C_{1-4}$-Alkylgruppe oder eine Ar($C_{1-4}$)alkylgruppe ist, X ein verträgliches Anion ist und n 2 oder 3 ist.

3. Verwendung einer Verbindung gemäß Anspruch 1 oder 2, worin in der allgemeinen Formel I X ein Halogenid, insbesondere Chlorid, Nitrat, Sulfat, Alkylsulfat, insbesondere Ethylsulfat, oder Alkylphosphatanion ist, und L einen Acylrest, der sich von raffinierter Lanolinsäure ableitet, darstellt.

4. Verwendung einer Verbindung gemäß Anspruch 1, worin die Desinfektionszusammensetzung als einen aktiven Bestandteil eine Mischung von Verbindungen gemäß der allgemeinen Formel I enthält.

5. Verwendung einer Verbindung gemäß einem der Ansprüche 1 bis 4, worin die Gesamtkonzentration der aktive Verbindung(en) im Bereich von 10.000 bis 50.000 p.p.m., insbesondere 10.000 bis 40.000 p.p.m., bezogen auf das Gewicht der Zusammensetzung liegt.

6. Verwendung einer Verbindung gemäß einem der Ansprüche 1 bis 5, worin die Desinfektionszusammensetzung wenigstens ein in Wasser lösliches oder emulgierbares Erweichungsmittel in einer Konzentration im Bereich von 0,1 bis 1,0%, insbesondere 0,25 bis 0,75%, bezogen auf das Gewicht der Zusammensetzung einschließt.

7. Verwendung einer Verbindung gemäß Anspruch 6, worin das Erweichungsmittel ausgewählt ist aus alkoxyliertem Lanolin, synthetischem Gefiederöl (welches ein hochverzweigter langkettiger Fettsäureester mit einer eine gerade Anzahl an Kohlenstoff enthaltenden Kohlenstoffkette im Säureteil des Moleküls oder eine Mischung solcher Ester ist), und ethoxyliertem synthetischen Gefiederöl der vorherbeschriebenen Art ausgewählt ist.

8. Verwendung einer Verbindung gemäß einem der Ansprüche 1 bis 7, worin die Desinfektionszusammensetzung ein oder mehrere quaternäre Ammoniumhalogenid verbindungen in einer Konzentration im Bereich von 0,010 bis 2,0 Gew.-% der Zusammensetzung für eine erhöhte biozide Aktivität einschließt.

9. Verwendung einer Verbindung gemäß Anspruch 8, worin die quaternäre Ammoniumhalogenidverbindung ausgewählt ist aus Benzalkoniumchlorid, Octyldecyldimethylammoniumchlorid, Dioctyldimethylammoniumchlorid und Didecyldimethylammoniumchlorid.

**Revendications**

1. Utilisation d'un composé de la formule générale I:

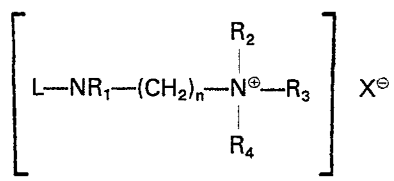

I

dans laquelle L représente un radical acyle provenant d'un acide gras de lanoline, $R_1$ représente de l'hydrogène ou un groupe alkyle en $C_1$—$C_4$, $R_2$ et $R_3$ représentent chacun un groupe alkyle en $C_1$—$C_4$, $R_4$ représente un radical alkyle, aralkyle, hydroxyalkyle ou hydrocarburé aliphatique insaturé, X représente un anion compatible et n est un nombre entier de 2 à 5, pour la fabrication d'une composition désinfectante pour le trempage de mamelons.

2. Utilisation d'un composé suivant la revendication 1, où dans la formule générale I, $R_1$ représente de l'hydrogène, $R_2$ et $R_3$ représentent indépendamment l'un de l'autre un groupe méthyle ou éthyle, $R_4$ représente un groupe alkyle en $C_1$—$C_4$ ou un groupe Ar($C_1$—$C_4$)alkyle, X représente un anion compatible n est égal à 2 ou 3.

3. Utilisation d'un composé suivant l'une ou l'autre des revendications 1 et 2, où dans la formule générale I, X représente un anion halogénure, en particulier chlorure, nitrate, sulfate, alkylsulfate, en particulier éthylsulfate ou alkylphosphate et L représente un radical acyle provenant d'un acide de lanoline raffiné.

4. Utilisation d'un composé suivant la revendication 1, caractérisée en ce que la composition désinfectante contient comme ingrédient actif un mélange de composés de formule générale I.

5. Utilisation d'un composé suivant l'une quelconque des revendications 1 à 4, caractérisée en ce que la concentration totale de composé(s) actif(s) est de l'ordre de 10.000 à 50.000 p.p.m., en particulier de 10.000 à 40.000 p.p.m. en poids de la composition.

6. Utilisation d'un composé suivant l'une quelconque des revendications 1 à 5, caractérisée en ce que la composition désinfectante comprend au moins un émollient, soluble ou émulsifiable dans l'eau, à une concentration de l'ordre de 0,1 à 1,0%, en particulier de 0,25—0,75% en poids de la composition.

7. Utilisation d'un composé suivant la revendication 6, caractérisée en ce que l'émollient est choisi parmi les lanolines alcoxylées, l'huile de plumage synthétique (qui est un ester gras à longue chaîne fortement ramifiée comportant une chaîne d'atomes de carbone en nombre pair dans la partie acide de la molécule ou un mélange de tels esters) et l'huile de plumage synthétique éthoxylée telle que définie précédemment.

8. Utilisation d'un composé suivant l'une quelconque des revendications 1 à 7, caractérisée en ce que la composition désinfectante comprend un ou plusieurs composés d'halogénure d'ammonium quaternaire à une concentration de l'ordre de 0,010—2,0% en poids de la composition, pour une activité biocide accrue.

9. Utilisation d'un composé suivant la revendication 8, caractérisée en ce que le composé d'halogénure d'ammonium quaternaire est choisi parmi le chlorure de benzalkonium, le chlorure d'octyl décyl diméthyl ammonium, le chlorure de dioctyl diméthyl ammonium et le chlorure de didécyl diméthyl ammonium.